(19) Europäisches Patentamt
European Patent Office
Office européen des brevets

(11) Veröffentlichungsnummer: 0 227 988
A2

(12) # EUROPÄISCHE PATENTANMELDUNG

(21) Anmeldenummer: 86117109.8

(22) Anmeldetag: 09.12.86

(51) Int. Cl.⁴: **A 61 L 15/03**

(30) Priorität: 14.12.85 DE 3544311

(43) Veröffentlichungstag der Anmeldung: 08.07.87 Patentblatt 87/28

(84) Benannte Vertragsstaaten: AT BE CH DE ES FR GB GR IT LI LU NL SE

(71) Anmelder: BOEHRINGER INGELHEIM KG, D-6507 Ingelheim am Rhein (DE)

(84) Benannte Vertragsstaaten: BE CH DE ES FR GR IT LI LU NL SE AT

(71) Anmelder: Boehringer Ingelheim International G.m.b.H, D-6507 Ingelheim am Rhein (DE)

(84) Benannte Vertragsstaaten: GB

(72) Erfinder: Brantl, Victor, Dr. med., Matthias-Claudius-Strasse 9, D-6200 Wiesbaden (DE)
Erfinder: Häselbarth, Volkmar, Dr. Dipl.-Chem., Im Hippel 53, D-6535 Gau-Algesheim (DE)
Erfinder: Zierenberg, Bernd, Dr. Dipl.-Chem., Goethestrasse 1, D-6530 Bingen/Rhein (DE)

(54) **Therapeutisches System.**

(57) Die vorliegende Erfindung betrifft die transdermale Applikation von Verbindungen der allgemeinen Formel I,

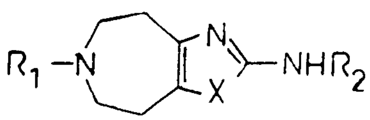

I

worin $R_1$, $R_2$ und X die im Text angegebene Bedeutung haben sowie ein therapeutisches System zur transdermalen Applikation dieser Verbindung.

EP 0 227 988 A2

Beschreibung

Die Erfindung betrifft die Verwendung von Verbindungen der allgemeinen Formel

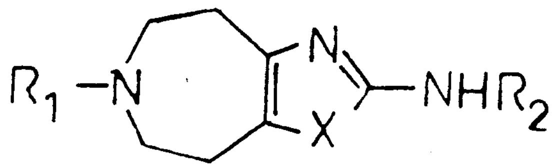

I

deren Säureadditionssalze mit physiologisch verträglichen anorganischen oder organischen Säuren, worin $R^1$ ein Wasserstoffatom, einen gegebenenfalls durch eine Hydroxylgruppe substituierten geradkettigen oder verzweigten Alkylrest mit 1 - 4 Kohlenstoffatomen, einen Allyl-, Cycloalkyl-, Hexahydrobenzyl-, Phenyl-, Phenylethyl- oder Benzylrest, wobei der Benzylrest im Kern durch ein oder zwei Halogenatome, durch ein bis drei Methoxygruppen, durch eine Trifluormethyl- oder Alkylgruppe mit 1 - 3 Kohlenstoffatomen substituiert sein kann und falls

X ein Schwefelatom darstellt,

$R^2$ ein Wasserstoffatom, einen geradkettigen oder verzweigten Alkylrest mit 1 - 5 Kohlenstoffatomen, einen Allyl-, Cycloalkyl-, Phenyl-, Benzyl- oder Phenylethylrest oder falls X ein Sauerstoffatom darstellt, ein Wasserstoffatom bedeutet,

zur transdermalen Applikation bei der Behandlung von Krankheiten.

Bevorzugte Verbindungen sind 2-Amino-6-ethyl-4,5,7,8-tetrahydro- 6H-oxazolo[5,4-d]azepin und 6-Allyl-2-amino-4,5,7,8-tetrahydro-6H-thiazolo[5,4-d]azepin sowie gegebenenfalls deren Säureadditionssalze.

Die Erfindung betrifft weiterhin ein therapeutisches System zur Abgabe eines Wirkstoffes durch die Haut über einen längeren Zeitraum, insbesondere von Verbindungen der allgemeinen Formel I, insbesondere von 2-Amino-6-ethyl-4,5,7,8-tetrahydro-6H-oxazolo[5,4-d]azepin, wie auch von 6-Allyl-2-amino-4,5,7,8-tetrahydro-6H-thiazolo[5,4-d]azepin sowie gegebenenfalls ihrer Säureadditionssalze.

Die pharmakologischen Eigenschaften dieser namentlich genannten Substanzen sowie ihrer Derivate sind aus der deutschen Offenlegungsschrift 20 40 510 bekannt, wobei sowohl hustenstillende wie auch blutdrucksenkende Eigenschaften beschrieben sind. Eine Retardform zur oralen Behandlung der Hypertonie und von Angina Pectoris ist in der DE-OS 28 36 387 offenbart.

Weiterhin sind das 2-Amino-6-ethyl-4,5,7,8-tetrahydro-6H-oxazolo[4,5-d]azepin wie auch das 6-Allyl-2-amino-4,5,7,8-tetrahydro-6H-thiazolo[5,4-d]azepin als B-HT 933 bzw. B-HT 920 in zahlreichen wissenschaftlichen Publikationen beschrieben.

Bislang war es war es nicht bekannt, die aus der DE-OS 20 40 510 bekannten Verbindungen, insbesondere 2-Amino-6-ethyl- 4,5,7,8-tetrahydro-6H-oxazolo[5,4 -d]-azepin wie auch 6-Allyl-2-amino-4,5,7,8-tetrahydro-6H-thiazolo[5,4-d]azepin transdermal zu applizieren, wodurch die bei der oralen Therapie auftretenden Nebenwirkungen, wie z.B. Mundtrockenheit oder Sedation vermieden werden können.

Der Erfolg der transdermalen Therapie hängt von unterschiedlichen Faktoren ab. Zum einen muß der Wirkstoff in ausreichend hoher Konzentration in dem therapeutischen System speicherbar sein, um ein Wirkstoffdepot zu bilden, andererseits kann nur eine kontrollierte Freigabe und eine ungehinderte Penetration durch die Haut einen therapeutisch wirksamen Blutspiegel im Körper aufbauen. Eine weitere Voraussetzung ist, daß der Wirkstoff weder allein noch in Kombination mit dem therapeutischen System die Haut schädigt, d.h. zu Irritationen, Allergien oder Sensibilisierungen führt.

Die erfindungsgemäß verwendeten Verbindungen, insbesondere 2-Amino-6-ethyl-4,5,7,8-tetrahydro-6H-oxazolo[5,4-d]azepin und 6-Allyl-2-amino-4,5,7,8-tetrahydro-6H-thiazolo-[5,4-d]azepin erfüllen unerwarteterweise diese Voraussetzungen.

Es sind bislang zahlreiche therapeutische Systeme in Form von Hautpflastern oder Bandagen bekannt geworden, die für eine transdermale Applikation von Wirkstoffen, bevorzugt von Nitroglycerin, geeignet sind. So ist z.B. aus der DE-PS 21 35 533 ein Haftverband zur Applikation von systemisch wirkenden Arzneimitteln beschrieben, der als charakteristisch notwendiges Merkmal eine Diffusionsmembrane zur Steuerung der Wirkstofffreisetzung zwischen der wirkstoffhaltigen Vorratsschicht und der Klebemittelschicht enthält.
Weiterhin ist aus der europäischen Patentschrift 33 615 bekannt, daß die steuernde Funktion der Membrane auch von der Kontaktklebefläche übernommen werden kann. Therapeutische Systeme, die den Wirkstoff in Form von mikroverkapselten Teilchen enthalten, sind beispielsweise aus der US-PS 3 742 951 bekannt.

In Anbetracht der Erfordernisse an die Arzneimittelsicherheit dürfen sich die Eigenschaften eines transdermalen therapeutischen Systems in Bezug auf das Freigabeverhalten und die Stabilität des Wirkstoffes auch nach einem längeren Zeitraum (z.B. durch Lagerung) nicht verändern.

Entgegen den Erwartungen zeigt das nach dem in der DE-OS 32 04 551 offenbarten Verfahren, aus einem gefriergetrockneten Latex aus einem Copolymerisat von Methyl- und/oder Ethylestern der Acryl- und Methacrylsäure und dem Wirkstoff, beispielsweise 2-Amino-6-ethyl-4,5,7,8-tetrahydro-6H-oxazolo[5,4-d]azepin, hergestellte therapeutische System nicht die erforderliche Stabilität. Bereits nach kurzer Zeit deutete eine bräunliche Verfärbung auf eine Zersetzung des Wirkstoffes hin, so daß die Verwendung als Arzneimittel-Zubereitung nicht mehr akzeptiert werden konnte. Ein ähnliches Verhalten ist bei der Verwendung von 6-Allyl-2-amino-4,5,7,8-tetrahydro-6H-thiazolo[5,4-d]azepin zu beobachten.

Diese Nachteile werden überraschenderweise durch das erfindungsgemäße therapeutische System vermieden.

Es wurde nun gefunden, daß sich die zuvor geschilderten Stabilitätsprobleme dadurch lösen lassen, daß die wirkstoffhaltige Reservoirschicht aus einem Emulsionspolymerisat oder -copolymerisat und einem Copolymerisat auf der Basis von Methacrylsäureestern mit basischen Endgruppen oder aber einem geringen Anteil einer organischen stickstoffhaltigen Base besteht.

Ein weiterer Gegenstand der vorliegenden Erfindung ist daher ein therapeutisches System in Form eines Hautpflasters aus einer wirkstoffundurchlässigen Rückschicht, einer wirkstoffhaltigen Reservoirschicht,

einem abzuziehenden Schutzfilm und Mitteln zur Befestigung auf der Haut, wobei die wirkstoffhaltige Reservoirschicht aus einem Emulsionspolymerisat oder -copolymerisat und einem Copolymerisat auf der Basis von Methacrylsäurestern mit basischen Endgruppen besteht, oder aber anstelle der basischen Endgruppen einen geringen Anteil einer organischen stickstoffhaltigen Base aufweist.

Bevorzugte Wirkstoffe sind Verbindungen der allgemeinen Formel I, besonders bevorzugt ist 2-Amino-6-ethyl-4.5.7.8-tetrahydro-6H-oxazolo[5,4-d]azepin und 6-Allyl-2-amino-4,5,7,8-tetrahydro-6H-thiazolo-[5,4-d]azepin.

Bevorzugte Emulsionspolymerisate der erfindungsgemäßen Ausführungsform des therapeutischen Systems sind PVC, Polylactide, Polysterol, Polyvinylacetat, Polybutadien, Polyacrylnitril, Polyvinylester, Polyvinylether sowie deren Copolymere.

Besonders bevorzugt sind emulsionspolymerisierte Copolymerisate von Methyl- und/oder Ethylestern der Acryl- und Methacrylsäure.

Das bevorzugte Copolymerisat mit basischen Endgruppen ist ein Copolymerisat auf der Basis von Dimethylaminoethylmethylacrylat und neutralen Methacrylsäureestern der allgemeinen Struktur mit den Untereinheiten A und B.

A

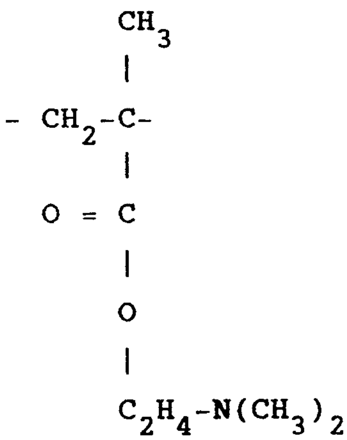

B

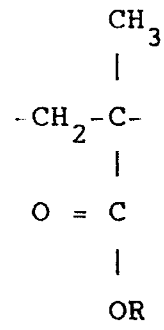

R = $CH_3$, $C_4H_9$

mit einem mittleren Molekulargewicht von ca. 150 000, wie es beispielsweise von der Firma Röhm, Darmstadt unter der Bezeichnung Eudragit® E 100 vertrieben wird.

Gemäß der Erfindung beträgt der Anteil des Copolymerisats mit den basischen Endgruppen zwischen 1 und 50 Gew.-%, bevorzugt 5 - 10 Gew.-%, bezogen auf den gesamten Polymeranteil der Reservoirschicht.

Zur Stabilisierung des Wirkstoffes kann der Reservoirschicht anstelle eines Copolymerisates mit basischen Endgruppen auch 0.5 - 5.0 Gew.-% , bezogen auf die Wirkstoffmenge, einer geeigneten, für die transdermale Applikation unbedenkliche organische Base zugesetzt sein.

Bevorzugt sind organische Basen der allgemeinen Formel

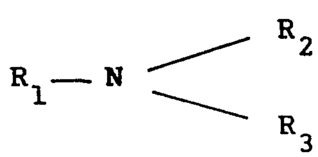

in der $R_1$, $R_2$ und $R_3$ unabhängig voneinander Wasserstoff, eine verzweigte oder unverzweigte Alkyl- oder Hydroxyalkylgruppe mit 1 bis 5 Kohlenstoffatomen bedeuten können Besonders bevorzugt sind Isopropylamin, ganz besonders bevorzugt sind Triethanolamin und Tris(hydroxymethyl)-aminomethan.

Die Wirkstoffkonzentration in der Reservoirschicht sollte im Bereich zwischen 0.5 und 6 mg, bevorzugt zwischen 1 und 4 mg pro $cm^2$ bei einer Schichtdicke der Reservoirschicht zwischen 40 und 300 µm, bevorzugt zwischen 50 und 200 µm, liegen.

Wenn es gewünscht wird, können sogenannte Lösungsvermittler (enhancer) zugesetzt werden, die die Diffusion des Arzneimittelwirkstoffes durch die Haut begünstigen. Bevorzugt verwendbare Verbindungen sind: Dimethyllauramid, 1-Dodecylazocycloheptan-2-on, Glyceroldimethylketal, Isopropylmyristat sowie N,N,Diethyl-m-toluamid (J. Pharm. Sci., Vol. 71, 1211 (1982)).

Die wirkstoffundurchlässige Rückschicht des erfindungsgemäßen Systems besteht vorzugsweise aus einem Laminat aus einer Metallfolie, wie z.B. Aluminium und einem Polymerfilm. Bevorzugte Polymere sind beispielsweise Hoch- und Niederdruckpolyethylen, Polypropylen, Polyvinylchlorid und Polyethylenterephthalsäure.

In einer besonderen Ausführungsform des erfindungsgemäßen Systems ist die wirkstoffundurchlässige Rückschicht so ausgestaltet, daß ihre Fläche größer ist als die der Reservoirschicht und ihre Unterseite mit einem physiologisch verträglichen Klebemittel beschichtet ist. Eine derart ausgestaltete Rückschicht dient gleichzeitig zur Fixierung der wirkstoffhaltigen Reservoirschicht und zur Befestigung des Systems auf der Haut. Geeignete Klebemittel sind beispielsweise Acrylharzdispersionen, wie z.B. Plex® 4853 der Firma Röhm, Darmstadt.

Gegebenenfalls kann zwischen der wirkstoffhaltigen Reservoirschicht und der Rückschicht eine wirkstoffundurchlässige Stützschicht aus einem Laminat aus einer dünnen Aluminiumfolie und einem Polyäthylenfilm angebracht sein. In diesem Fall ist die Rückschicht von größerer Fläche als die des Reservoirs und aus einem Gewebe oder Vlies, dessen Rückseite mit einem Klebemittel beschichtet ist.
In einer weiteren Ausführungsform kann das erfindungsgemäße System aus einer wirkstoffundurchlässigen Rückschicht, einer Reservoirschicht und einer Klebemittelschicht bestehen. Klebemittel, die eine ungehinderte Diffusion des Wirkstoffs ermöglichen, sind aus dem Stand der Technik bekannt.

Die Wirkstofffreigabe des erfindungsgemäßen Systems kann durch geeignete Auswahl physikalischer Parameter der verwendeten Emulsionspolymerisate gesteuert werden. Hierzu gehören die Variation der Glastemperatur durch eine entsprechende Auswahl der Monomerenzusammensetzung und die Variation der Teilchengröße der Polymerteilchen, die durch die Reaktionsbedingungen bei der Polymerisation eingestellt werden kann.

Ein Anstieg der Glastemperatur ist mit einer Erniedrigung der Freisetzungsrate, eine Abnahme der Teilchengröße mit einer Erhöhung der Freisetzungsrate verbunden.

Die Möglichkeit, die Wirkstofffreigabe des zuvor beschriebenen therapeutischen Systems durch die oben beschriebenen Parameter in einem weiten Bereich frei einstellen zu können, erlaubt eine optimale Anpassung an die zu behandelnde Indikation.

Wie bereits ausgeführt wurde, ist die Herstellung von Verbindungen der allgemeinen Formel I, insbesondere von 2-Amino-6-ethyl-4,5,7,8-tetrahydro-6H-oxazolo[5,4-d]-azepin, 6-Allyl-2-amino-4,5,7,8-tetrahydro-6H-thiazolo-[5,4-d]azepin wie auch ihrer Säureadditionssalze aus der DE-OS 20 40 510 bekannt. Bezüglich der Herstellung dieser Verbindungen wird auf die Offenbarung in der genannten Offenlegungsschrift verwiesen.

Das therapeutische System führt den Wirkstoff transdermal dem Blutkreislauf zu und bewirkt eine zentrale α-Stimulation, die im Fall, daß der Wirkstoff 2-Amino-6-ethyl-4,5,7,8-tetrahydro-6H-oxazolo[5,4-d]azepin ist, zu einer vorbeugenden oder heilenden Behandlung des Bluthochdrucks, der Migräne, Beschwerden des Postmenopause, Menstruationsbeschwerden oder Angina Pectoris führt. Weiterhin ist es zur Behandlung von Entzugssymptomen, wie z.B. bei Nikotinentzug, geeignet. Ein bevorzugtes Anwendungsgebiet ist die Behandlung des Bluthochdrucks durch die transtermale Applikation von B-HT 933. Im Fall, daß der Wirkstoff 6-Allyl-2-amino-4,5,7,8-tetrahydro-6H-thiazolo[5,4-d]azepin ist, sind die bevorzugten Indikationen die Senkung des Prolactin-Spiegels, die Behandlung der Parkinsonschen Erkrankung und die Behandlung der Schizophrenie.

Unerwünschte Nebenwirkungen, wie sie bei einer oralen Therapie auftreten, wie z.B. starke Mundtrockenheit oder Sedation, werden nicht beobachtet bzw. treten nur in stark abgeschwächter Form in Erscheinung.
Hautunverträglichkeiten oder Irritationen, wie sie auftreten können, wenn chemische Verbindungen über einen längeren Zeitraum auf ein und dieselbe Hautstelle aufgebracht werden, werden weder im Tierexperiment, noch in klinischen Prüfungen am Menschen beobachtet.

Das Fehlen solcher Nebeneffekte bei der transdermalen Applikation von 2-Amino-6-ethyl-4,5,7,8-tetrahydro-6H-oxazolo[5,4-d]azepin ist ein großer Vorteil bei der Behandlung von Wachstumsstörungen bei Kindern und Jugendlichen.Ein weiterer Gegenstand der vorliegenden Erfindung ist demnach die Verwendung von B-HT 933 zur Herstellung eines Arzneimittels zur Behandlung von Wachstumsstörungen. B-HT 933, bzw. dessen Säureadditionssalze kann zur Behandlung von Krankheiten eingesetzt werden, die auf einer verminderten Ausschüttung des Wachstumshormons beruhen, so z.B. bei Minderwuchs, ebenso bei vermindertem Stoffwechsel, wie z.B. Maluntrition, Kachexie bei Tumoren oder der Chemotherapie; chronische Anoxie durch respiratorische Insuffizienz oder Cardiopathie, Niereninsuffizienz. Weitere Indikationsgebiete sind Knochenfrakturen, Verbrennungen, Wundheilung und Beschleunigung der Blutbildung.

Zu diagnostischen Zwecken kann B-HT 933 eingesetzt werden, um die Freisetzung von Wachstumshormon zu stimulieren und um so festzustellen, ob ausreichend Wachstumshormon in der Hypophyse vorhanden ist.

Arzneimittel zur Behandlung der genannten Indikation (Wachstumsstörungen) können nicht nur in Form transdermaler Systeme hergestellt werden, sondern ebenfalls in Form von Tabletten, Lösungen, Suspensionen, Zäpfchen etc. Bezüglich der Herstellung galenischer Darreichungsformen wird auf die deutschen Offenlegungsschriften 20 40 510 und 28 36 387 verwiesen. Die therapeutisch wirksame Einzeldosis liegt bei nicht transdermaler Applikation zwischen 2,5 und 50, bevorzugt zwischen 5 bix 15 mg je Dosiseinheit.

Eine bevorzugte Ausführungsform des beschriebenen therapeutischen Systems kann nach folgendem Verfahren hergestellt werden:

Das emulsionspolymerisierte Copolymerisat mit einem Anteil an basischen Endgruppen wird zusammen mit dem Wirkstoff in einem geeigneten Lösungsmittel gelöst, wobei eine viskose bis hochviskose Dispersion entsteht. Enthält das Copolymerisat keine basischen Endgruppen, kann eine entsprechende Menge einer organischen stickstoffhaltigen Base zur Stabilisierung des Wirkstoffes zugesetzt werden. Als Lösungsmittel kommen niedere aliphatische Alkohole, Ether, Ketone, Ester, Kohlenwasserstoffe bzw. Halogenkohlenwasserstoffe in Frage, insbesondere solche, deren Siedepunkt unter 100°C liegt und die sich leicht verdampfen lassen. Auch Lösungsmittelgemische sind verwendbar. Durch Wahl eines geeigneten Lösungsmittels oder Lösungsmittelgemisches lassen sich die Viskositäten der Ausgangslösungen verändern.
Nachdem eine homogene Lösung (Dispersion) entstanden ist, wird diese auf die vorbereitete Stützschicht aus einem Laminat aus einer Aluminiumfolie und einer Polyethylenfolie ausgegossen, so daß die Schichtdicke der Wirkstoffschicht nach der Trocknung zwischen 50 und 200 µm beträgt. Die Trocknung erfolgt normalerweise bei Raumtemperatur, oder leicht erhöhter Temperatur, gegebenenfalls kann unter vermindertem Druck getrocknet werden. Soweit es sich um lichtempfindliche Verbindungen handelt, kann die Herstellung des therapeutischen Systems unter Lichtausschluß erfolgen.

Die Filmherstellung kann auch kontinuierlich auf einer Walzenstraße erfolgen.

Anschließend wird der so hergestellte Film mit einer abziehbaren Schutzschicht versehen und konfektioniert.

In einer weiteren Ausführungsform kann die Dispersion auch direkt auf eine entsprechend vorbereitete Rückschicht aufgegossen werden.

Die folgenden Beispiele sollen die Erfindung verdeutlichen, ohne sie jedoch einzuschränken.

Herstellungsbeispiel 1:

Zusammensetzung:

| | | |
|---|---|---|
| 9.744 | g | Eudragit® E 30 D (Fa. Röhm, Darmstadt) |
| 0.600 | g | Eudragit® E 100 (Fa. Röhm, Darmstadt) |
| 1.656 | g | 2-Amino-6-ethyl-4,5,7,8-tetrahydro-6H-oxazolo[5,4-d]azepin |
| 12.000 | g | Feststoff |
| 6.000 | g | Aceton |
| 22.000 | g | Methanol |
| 100.000 | g | Lösung |

Das Eudragit® E 100 wird in Aceton vorgelöst, anschließend wird das Eudragit® 30 D und die Hälfte des Methanols zugegeben und gerührt. Nachdem eine homogene Lösung entstanden ist, wird der Wirkstoff und das restliche Methanol portionsweise eingetragen. Die so erhaltene Lösung wird auf ein Filmziehgerät (d. Fa. Erichsen) auf eine vorbereitete Rückschicht gegossen und bei einer Rakelstellung von 0.6 mm abgezogen. Nach 10 minütigem Trocknen wird eine weitere und dann noch eine dritte Schicht bei gleicher Rakelstellung aufgetragen. Nach dem Trocknen erhält man einen Film mit einer Schichtdicke von 100 µm.

Herstellungsbeispiel 2

Zusammensetzung:

| | | |
|---|---|---|
| 9.144 | g | Eudragit® E 30 D |
| 1.200 | g | Eudragit® E 100 |
| 1.656 | g | 2-Amino-6-ethyl-4,5,7,8-tetrahydro-6H-oxazolo[5,4-d]azepin |
| 12.000 | g | Feststoff |
| 66.000 | g | Aceton |
| 22.000 | g | Methanol |
| 100.000 | g | Lösung |

Die Herstellung des Films erfolgte analog dem Beispiel 1.

Herstellungsbeispiel 3

Zusammensetzung:

| a | | b | | |
|---|---|---|---|---|
| 10.000 | g | 10.620 | g | Eudragit® E 30 D |
| 0.300 | g | 0.280 | g | Eudragit® E 100 |
| 0.800 | g | 1.000 | g | Wirkstoff |
| 12.000 | g | 12.000 | g | Feststoff |
| 53.000 | g | 53.000 | g | Aceton |
| 10.000 | g | 10.000 | g | Methanol |

Die Herstellung der Filme erfolgte analog dem Beispiel 1, jedoch bei einer Rakelstellung von 0.98 mm. Die Filmstärke betrug 200 µm.

Herstellungsbeispiel 4:

Zusammensetzung:

| A | B | |
|---|---|---|
| 7.533 g | 5.022 g | Eudragit® E 30 D |
| 2.511 g | 5.022 g | Polyacrylat TG 29* |
| 0.300 g | 0.300 g | Triethanolamin |
| 1.656 g | 1.656 g | 2-Amino-6-ethyl-4,5,7,8-tetrahydro-6H-oxazolo[5,4-d]azepin |
| 12.000 g | 12.000 g | Feststoff |
| 53.000 g | 53.000 g | Aceton |
| 10.000 g | 10.000 g | Methanol |

Die Herstellung der Filme erfolgte analog dem in Beispiel 1 beschriebenen Verfahren, wobei jedoch nur eine zweimalige Dispersionsauftragung mit einer Rakelstellung von jeweils 0.8 mm erfolgte.

* Dieses Polyacrylat unterscheidet sich von Eudragit® E 30 D lediglich durch die Monomerenverteilung, wodurch eine höhere Glastemperatur von +29°C erhalten wird.

Herstellungsbeispiel 5:

Zusammensetzung:

| A | B | C | |
|---|---|---|---|
| 10.224 g | 10.044 g | 9.744 g | Eudragit® E 30 D |
| 0.120 g | 0.300 g | 0.600 g | Triethanolamin |
| 1.656 g | 1.656 g | 1.656 g | Wirkstoff* |
| 12.000 g | 12.000 g | 12.000 g | Feststoff |
| 53.000 g | 53.000 g | 53.000 g | Aceton |
| 10.000 g | 10.000 g | 10.000 g | Methanol |

Die Herstellung der Filme erfolgte analog dem Herstellungsbeispiel 4.

* Im vorstehenden Beispiel wurde als Wirkstoff sowohl 2-Amino-6-ethyl-4,5,7,8-tetrahydro-6H-oxazolo[5,4-d]azepin wie auch 6-Allyl-2-amino-4,5,7,8-tetrahydro-6H-thiazolo[5,4-d]azepin verwendet.

Herstellungsbeispiel 6:

Produktaufbau: runde Pflaster zu 10 $cm^2$, 2 $mg/Cm^2$, Schichtdicke 150 µm

a) Träger: PE/Alu/-Folie (1)
b) Wirkstoff-Polyacrylatschicht (2)
c) Deckpflaster mit Schutzpapier (3)
d) Folie wie a) als Einschweißbeutel

Auftragslösung

| | | |
|---|---|---|
| 10,631 g | Eudragit® E 30 D, luftgetrocknet | (88,59 %) |
| 1,333 g | B-HT 933 BS | (11,11 %) |
| 0,036 g | Tris-(hydroxymethyl)-aminomethan | ( 0,30 %) |
| 12,000 g | Feststoff | |
| 15,000 g | Methanol, p.a. | |
| 26,500 g | Aceton, p.a. | |
| 53,500 g | Lösung (22,4 %ig) | |

(1) PE = Polyethylen, Alu-Aluminium

(2) Luftgetrocknetes Eudragit® E 30 hergestellt aus wässriger Eudragit® E 30 Dispersion

(3) Deckpflaster hautfarben, 53 x 53 mm mit weißem Deckpapier.

Geräte

Magnetrührer, Erichsen-Filmziehgerät mit Rakel und Dunkelhaube, Stanzeisen mit 10 $cm^2$ Fläche, Folienschweißgerät.

Herstellung

Vorbereitung

Das Erichsen-Gerät wird mit Verbundfolie bespannt und mittels Vakuum plan festgesaugt.

Wirkstofflösung

Das Aceton und die Hälfte der Methanolmenge werden vorgelegt, das Tris(hydroxymethyl)-aminomethan gelöst und das Eudragit® E 30 D unter kräftigem Rühren eingetragen und bis zur vollkommenen Lösung gerührt. Das B-HT 933 BS wird in einem Teil der Restmethanolmenge gelöst, eingerührt und mit dem anderen Teil nachgespült.

Filme

Die Lösung wird einmal mit Rakelstellung ca. 1,4 mm aufgetragen. Nach dem Trocknen erhält man einen wirkstoffhaltigen Film mit einer Beschichtungsmenge von ca. 18,0 $mg/cm^2$ entsprechend einer Schichtdicke von ca. 150 µm.

Ausstanzen und Abpacken

Die Filmplättchen werden umgehend ausgestanzt, in die Deckpflaster verpackt und in vorbereitete Beutel aus AL/PE-Folie mit etwas Stickstoffbegasung eingesiegelt.

Die Bestimmung der in vitro-Wirkstofffreigabe erfolgte in einem modifizierten USP-XVII Tester; hierzu wurden ca. 3 $cm^2$ große auf der Rückseite abgedeckte Filmstücke in 10 ml 32°C warmes demineralisiertes Wasser gelegt, das leicht durchmischt und in entsprechenden Zeitabständen analysiert wurde.

Die folgende Tabelle zeigt die abgegebene Wirkstoffmenge nach 8, 24 und 48 Stunden in mg und % an.

Freigabe des Films, hergestellt nach dem Herstellungsbeispiel 1:

| Versuch Nr. | 8 Stunden | 24 Stunden | 48 Stunden | |
|---|---|---|---|---|
| 1 | 3.1743 | 3.9488 | 4.1642 | mg |
| | 73.85 | 91.87 | 96.88 | % |
| 2 | 3.2186 | 4.0603 | 4.3036 | mg |
| | 72.61 | 91.59 | 97.08 | % |
| 3 | 2.4225 | 3.6707 | 4.0083 | mg |
| | 57.97 | 87.83 | 95.91 | % |
| Mittelwert | 2.9385 | 3.8933 | 4.1587 | mg |
| | 68.14 | 90.43 | 96.63 | % |

Freigabe des Films, hergestellt nach dem Herstellungsbeispiel 2.

| Versuch Nr. | 8 Stunden | 24 Stunden | 48 Stunden | |
|---|---|---|---|---|
| 1 | 2.5128 | 4.1762 | 4.5720 | mg |
| | 52.75 | 87,67 | 95,97 | % |
| 2 | 2.4041 | 4.0224 | 4.4049 | mg |
| | 52.38 | 87.65 | 95.98 | % |
| 3 | 2.4312 | 3.9532 | 4.3255 | mg |
| | 54.02 | 87.83 | 96.11 | % |
| Mittelwert | 2.4494 | 4.0506 | 4.4341 | mg |
| | 53.05 | 87,72 | 96,02 | % |

Freigabe des Films, hergestellt nach dem Herstellungsbeispiel 3a.

| Versuch-Nr. | 8 Stunden | 24 Stunden | 48 Stunden | |
|---|---|---|---|---|
| 1 | 0.4028 | 0.9133 | 1.3914 | mg |
| | 15.05 | 34.13 | 51.99 | |
| 2 | 0.4153 | 1.2854 | 1.9628 | mg |
| | 13.02 | 40.29 | 61.52 | % |
| 3 | 0.4272 | 0.9261 | 2.1449 | mg |
| | 11.64 | 25.23 | 58.44 | % |
| Mittelwert | 0.4165 | 1.0281 | 1.9253 | mg |
| | 12.90 | 31,90 | 58.56 | % |

Freigabe des Films, hergestellt nach dem Herstellungsbeispiel 3b.

| Versuchs-Nr. | 8 Stunden | 24 Stunden | 48 Stunden | |
|---|---|---|---|---|
| 1 | 0.7576 | 1.9396 | 2.5619 | mg |
| | 21.00 | 53.76 | 71.01 | % |
| 2 | 0.7467 | 2.0123 | 2.7526 | mg |
| | 19.11 | 51.50 | 70.45 | % |
| 3 | 0.7380 | 2.4632 | 3.8498 | mg |
| | 16.42 | 54.79 | 85.63 | % |
| 4 | 0.7356 | 1.8289 | 2.4715 | mg |
| | 19.02 | 47.28 | 63.89 | % |
| Mittelwert | 0.7445 | 2.0610 | 2.9090 | mg |
| | 18.89 | 51.83 | 72.75 | % |

Klinische Untersuchungen mit 2-Amino-6-ethyl-4,5,7,8-tetrahydro-6H-oxazolo[5,4-d]azepin als Wirkstoff.

Nach Applikation eines Pflasters mit einem Wirkstoffgehalt von 2,3 mg/2,5 $cm^2$ bei drei gesunden männlichen Probanden wurden die Plasmakonzentrationen des 2-Amino-6-ethyl-4,5,7,8-tetrahydro-6H-oxazolo[5,4-d]azepins in 24 Stunden-Intervallen gemessen

Pro Proband fielen 5 Plasmaproben in der Zeit 0, 24, 48, 72 und 96 Stunden nach Applikationsbeginn an.

Als analytische Methode wurde der RIA (Radioimmunoassays) verwendet, wobei von jeder vorliegenden Probe eine Doppelbestimmung durchgeführt wurde.
In Tabelle 1 sind alle gemessenen Einzelwerte zusammengestellt.

Tabelle 1

Konzentrationen (ng/ml) des Wirkstoffs in Plasma von 3 Probanden nach Applikation eines Pflasters mit 2,3 mg/ 2,5 $cm^2$ Wirkstoffgehalt.

Ergebnisse der Doppelbestimmungen durch RIA.

| | Probanden Nr. | | |
|---|---|---|---|
| Zeit | 1 | 2 | 3 |
| 0 h | 0.00 | 0.00 | 0.00 |
| | 0.00 | 0.00 | 0.00 |
| 24 h | 0.28 | 0.10 | 0.14 |
| | 0.31 | 0.08 | 0.13 |
| 48 h | 0.41 | 0.31 | 0.29 |
| | 0.41 | 0.30 | 0.31 |
| 72 h | 0.30 | 0.40 | 0.17 |
| | 0.28 | 0.35 | 0.16 |
| 96 h | 0.26 | 0.22 | 0.18 |
| | 0.26 | 0.22 | 0.18 |

Bei keinem der Probanden wurden Nebenwirkungen oder Hautunverträglichkeiten beobachtet.

## Patentansprüche

1) Therapeutisches System in Form eines Hautpflasters, bestehend aus einer wirkstoffundurchlässigen Rückschicht, einer wirkstoffhaltigen Reservoirschicht und Mitteln zur Befestigung auf der Haut zur transdermalen Applikation eines Wirkstoffes, dadurch gekennzeichnet, daß der Wirkstoff eine Verbindung der allgemeinen Formel

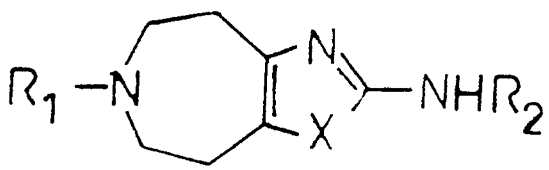

I

worin $R^1$ ein Wasserstoffatom, einen gegebenenfalls durch eine Hydroxylgruppe substituierten geradkettigen oder verzweigten Alkylrest mit 1 - 4 Kohlenstoffatomen, einen Allyl-, Cycloalkyl-, Hexahydrobenzyl-, Phenyl-, Phenylethyl- oder Benzylrest, wobei der Benzylrest im Kern durch ein oder zwei Halogenatome, durch ein bis drei Methoxygruppen, durch eine Trifluormethyl- oder Alkylgruppe mit 1 - 3 Kohlenstoffatomen substituiert sein kann und, falls

X ein Schwefelatom darstellt,

$R^2$ ein Wasserstoffatom, einen geradkettigen oder verzweigten Alkylrest mit 1 - 5 Kohlenstoffatomen, einen Allyl-, Cycloalkyl-, Phenyl-, Benzyl- oder

Phenylethylrest oder, falls X ein Sauerstoffatom darstellt, ein Wasserstoffatom bedeutet, bzw. Säureadditionssalze mit physiologisch verträglichen anorganischen oder organischen Säuren ist.

2) Therapeutisches System nach Anspruch 1, dadurch gekennzeichnet, daß der Wirkstoff 2-Amino-6-ethyl-4,5,7,8-tetrahydro-6H-oxazolo[5,4-d]-azepin oder dessen pharmakologisch verträgliches Säureadditionssalz ist.

3) Therapeutisches System nach Anspruch 1, dadurch gekennzeichnet, daß der Wirkstoff 6-Allyl-2-amino-4,5,7,8-tetrahydro-6H-thiazolo[5,4d]-azepin oder dessen pharmakologisch verträgliches Säureadditionssalz ist.

4) Therapeutisches System nach einem der Ansprüche 1 bis 3, dadurch gekennzeichnet, daß die Reservoirschicht aus einem Emulsionspolymerisat oder einem Emulsionscopolymerisat ausgewählt aus der Gruppe Polyvinylchlorid, Polylactid, Polysterol, Polyvinylacetat, Polybutadien, Polyacrylnitril, Polyvinylester, Methyl-, Ethylester der Acryl- und Methacrylsäure und gegebenenfalls aus einem Emulsionspolymerisat oder Emulsionscopolymerisat mit basischen Endgruppen mit einem Gewichtsanteil von 1 bis 50 % bezogen auf die Gesamtmenge an Polymerisat besteht.

5) Therapeutisches System nach Anspruch 4, dadurch gekennzeichnet, daß die Reservoirschicht 0.5 bis 5 Gew.-% bezogen auf die Wirkstoffmenge einer organischen stickstoffhaltigen Base enthält.

6) Verwendung von Verbindungen der allgemeinen Formel I

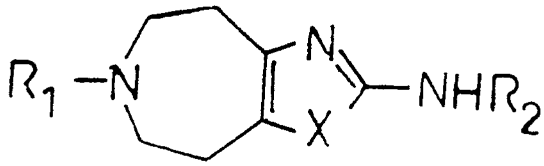

I

worin $R^1$ ein Wasserstoffatom, einen gegebenenfalls durch eine Hydroxylgruppe substituierten geradkettigen oder verzweigten Alkylrest mit 1 - 4 Kohlenstoffatomen, einen Allyl-, Cycloalkyl-, Hexahydrobenzyl-, Phenyl-, Phenylethyl- oder Benzylrest, wobei der Benzylrest im Kern durch ein oder zwei Halogenatome, durch ein bis drei Methoxygruppen, durch eine Trifluormethyl- oder Alkylgruppe mit 1 - 3 Kohlenstoffatomen substituiert sein kann und, falls

X ein Schwefelatom darstellt,

$R^2$ ein Wasserstoffatom, einen geradkettigen oder verzweigten Alkylrest mit 1 - 5 Kohlenstoffatomen, einen Allyl-, Cycloalkyl-, Phenyl-, Benzyl- oder Phenylethylrest oder, falls X ein Sauerstoffatom darstellt, ein Wasserstoffatom bedeutet, bzw. Säureadditionssalze mit physiologisch verträglichen anorganischen oder organischen Säuren ist, zur transdermalen Applikation bei der Behandlung von Krankheiten.

7) Verwendung von 2-Amino-6-ethyl-4,5,7,8-tetrahydro-6H-oxazolo [5,4-d]-azepin und dessen pharmakologisch verträgliche Säureadditionssalze zur transdermalen Applikation bei der Behandlung von Krankheiten.

8) Verwendung von 6-Allyl-2-amino-4,5,7,8-tetrahydro-6H-thiazolo[5,4-d]azepin und dessen pharmakologisch verträgliche Säureadditionssalze zur transdermalen Applikation bei der Behandlung von Krankheiten.

9) Verfahren zur Herstellung eines therapeutischen Systems in Form eines Hautpflasters zur transdermalen Applikation eines Wirkstoffes, dadurch gekennzeichnet, daß ein Emulsionspolymerisat oder Emulsionscopolymerisat, gegebenenfalls mit 1 bis 50 Gew.-% eines Emulsionspolymerisats mit basischen Endgruppen zusammen mit einem Wirkstoff der allgemeinen Formel

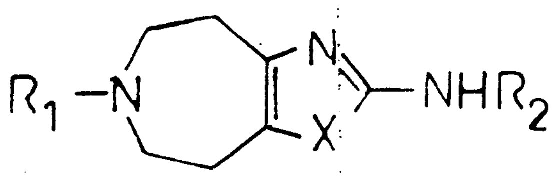 I

worin $R^1$ ein Wasserstoffatom, einen gegebenenfalls durch eine Hydroxylgruppe substituierten geradkettigen oder verzweigten Alkylrest mit 1 - 4 Kohlenstoffatomen, einen Allyl-, Cycloalkyl-, Hexahydrobenzyl-, Phenyl-, Phenylethyl- oder

Benzylrest, wobei der Benzylrest im Kern durch ein oder zwei Halogenatome, durch ein bis drei Methoxygruppen, durch eine Trifluormethyl- oder Alkylgruppe mit 1 - 3 Kohlenstoffatomen substituiert sein kann und, falls

X ein Schwefelatom darstellt,

$R^2$ ein Wasserstoffatom, einen geradkettigen oder verzweigten Alkylrest mit 1 - 5 Kohlenstoffatomen, einen Allyl-, Cycloalkyl-, Phenyl-, Benzyl- oder Phenylethylrest oder, falls X ein Sauerstoffatom darstellt, ein Wasserstoffatom bedeutet, bzw. Säureadditionssalze mit physiologisch verträglichen anorganischen oder organischen Säuren ist, in einem organischen Lösungsmittel gelöst, gegebenenfalls unter Zusatz von 1 bis 5 Gew.-%, bezogen auf die Wirkstoffmenge, einer organischen stickstoffhaltigen Base, zu einem Film ausgegossen und der Film nach dem Trocknen gegebenenfalls mit einer wirkstoffundurchlässigen Rückschicht und einer abziehbaren Schutzschicht versehen und konfektioniert wird.

10) Verwendung von 2-Amino-6-ethyl-4,5,7,8-tetrahydro-6H-oxazolo[5,4-d]azepin oder dessen pharmkologisch verträglichen Säureadditonssalze zur Herstellung eines Arzneimittels zur Behandlung von Wachstumsstörungen.

11) Verwendung von 2-Amino-6-ethyl-4,5,7,8-tetrahydro-6H-xazolo[5,4-d]azepin oder dessen pharmakologisch verträglichen Säureadditionssalze zur Herstellung eines Arzneimittels zur Freisetzung von Wachstumshormon.

12) Verwendung von 2-Amino-6-ethyl-4,5,7,8-tetrahydro-6H-oxazolo[5,4-d]azepin oder dessen verträgliche Säureadditionssalze zur Herstellung eines Arzneimittels zur Behandlung von Entzugssymptomen infolge von Drogenmißbrauchs.